(19) Europäisches Patentamt

European Patent Office

Office européen des brevets

(11) **EP 0 629 291 B1**

(12) **EUROPEAN PATENT SPECIFICATION**

(45) Date of publication and mention
of the grant of the patent:
**17.04.2002 Bulletin 2002/16**

(21) Application number: **93905515.8**

(22) Date of filing: **04.03.1993**

(51) Int Cl.[7]: **G01N 33/487**, A61B 5/05,
A61B 5/00

(86) International application number:
**PCT/GB93/00458**

(87) International publication number:
**WO 93/18402 (16.09.1993 Gazette 1993/22)**

(54) **ANALYTICAL OR MONITORING APPARATUS AND METHOD**

VORRICHTUNG UND VERFAHREN ZUR ANALYSE ODER ÜBERWACHUNG

APPAREIL ET PROCEDE D'ANALYSE OU DE CONTROLE

(84) Designated Contracting States:
**AT BE CH DE DK ES FR GB GR IE IT LI LU NL PT SE**

(30) Priority: **04.03.1992 GB 9204689**

(43) Date of publication of application:
**21.12.1994 Bulletin 1994/51**

(73) Proprietor: **The University of Wales, Aberystwyth
Aberystwyth, Dyfed SY23 3DA (GB)**

(72) Inventors:
• **KELL, Douglas Bruce
Aberystwyth, Dyfed SY23 3HA (GB)**

• **WOODWARD, Andrew Michael
Anglesey, Gwynedd LL61 6RS (GB)**

(74) Representative: **Cawdell, Karen Teresa
Urquhart-Dykes & Lord
Three Trinity Court
21-27 Newport Road
Cardiff, CF24 0AA (GB)**

(56) References cited:
**EP-A- 0 101 880      DE-A- 3 643 263
DE-U- 8 903 616      US-A- 4 240 027**

## Description

This invention relates to apparatus and method for analysis or monitoring of biological material and particularly, but not exclusively, to apparatus for monitoring or analysis of cellular biological material, in which a response is obtained from enzymes retained within cell membranes.

The linear passive audio and radio frequency electrical properties of biological cell materials are well known. In the frequency range below about 10 MHz, these properties are conveniently measured as the equivalent parallel conductance and capacitance of an electrochemical cell containing the system under study. Up to these radio-frequencies, most biological cell materials exhibit two major dispersions, known as the alpha and beta dispersions. Whilst other sub-dispersions contribute to these major dispersions, and may occasionally be separated from them, the beta dispersion of tissues and cell suspensions is caused predominantly by the build-up of charge at the essentially non-conducting plasma membrane surfaces. The alpha dispersion, though not always dependent upon the ionic strength of the medium, is usually accounted for mainly in terms of the relaxation of counter-ions tangential to the charged surfaces of the membrane and cell envelope.

In the simplest case of dielectric relaxation, that of the reorientation of a "hard" sphere with a permanent dipole moment, the statistical mean of the cosine of the angle which the dipole makes with the field, has a field-dependence following the Langevin function:-

$$\cos <\theta> = \coth x - 1/x,$$

where $x = E/kT$. A Taylor expansion of this series shows that substantial deviations from linearity do not occur for values of $x$ less than approximately 1, and that to an excellent approximation $\cos <\theta> = \mu E/3kT$. Thus the dielectric displacement current is proportional to the magnitude of the exciting field, and their ratio, the admittance, independent of it. These properties are characteristic of a linear system obeying the fluctuation-dissipation theorem.

Due to the fact that they are suspended or dissolved in conductive aqueous media, biological dielectrics are "lossy". Thus electrochemical reactions, and especially Joule heating, restrict the AC voltages that may be applied to them, and the dielectric properties of biological cell materials are typically measured using macroscopic electrical fields E of the order 0.1 to 5 V. $cm^{-1}$. Given the effective dipole moments usually encountered, the Langevin factor $\mu E/kT$ is normally minuscule, and, as judged by the independence of the measured admittance from the exciting field, well within the range of linearity.

It is known to measure the linear properties of biological materials using impedimetric instruments designed to filter out currents and voltages at frequencies other than the fundamental. This results in materials which appear to have linear characteristics but are in fact non-linear dielectrics.

We have described in GB-A-2247530 a method and apparatus for analysis of biological cell material, substrates therefor, or inhibitors of cell metabolism for cell material, the method comprising applying an AC electrical potential across a sample of the biological material so as to produce a non-linear dielectric spectrum, and obtaining a detectable signal corresponding to said spectrum.

The above GB-A-2247530 describes that, by applying a pure sinusoidal AC current to excite a biological cell material, if instead of observing the AC current only at the frequency applied, the entire frequency range of interest is observed, a non-linear dielectric spectrum may be produced from a biological material at voltage levels at which hitherto, the material has been expected to exhibit linear properties only.

The entire frequency range of interest may be studied by performing a transformation on the signal, for example a Fourier transform.

The apparatus described in GB-A-2247530, which (as previously indicated) is for analysing biological cell material, substrates therefor, or inhibitors of cell metabolism for cell material, comprises:

(a) retaining means for retaining a sample of biological material;
(b) means for applying an AC electrical potential across the sample so as to produce a non-linear dielectric spectrum; and
(c) means for obtaining a detectable signal corresponding to the spectrum.

The present invention provides apparatus for monitoring or analysing a determinand associated with cellular biological material, which apparatus comprises:

(a) means for applying an AC electrical potential at one or more discrete frequencies to cellular biological material;
(b) means for determining a response of the material at one or more frequencies which were substantially absent from the applied AC potential; and
(c) means for comparing the response to a calibration model of the determinand.

The determinand may be a concentration or other variable in cellular biological material (such as viable or living tissue in, for example, an animal, such as a human animal). An example of a preferred determinand is glucose concentration. The response obtained at one or more frequencies absent from the applied AC potential is referred to as a non-linear dielectric spectrum, as described in more detail in the above GB-A-2247530.

The apparatus according to the invention can be used, by way of example, in a method of monitoring the ability of living or viable cell material to transduce exogenous electric field energy. We have discovered that the particular harmonics present in the non-linear dielectric spectrum obtained from a cellular biological material are indicative of the metabolic state of living cells in the biological material.

The means for applying an AC potential preferably comprises a plurality of appropriate electrodes, a coil or the like, generally of known type; the electrodes are preferably arranged to be substantially flush with the skin of a patient so that the apparatus can be used for non-invasive monitoring of physiological parameters of the patient.

The response of the material at one or more frequencies which were substantially absent from the applied AC potential may be determines substantially as described in GB-A-2 247 530.

It is therefore not necessary according to the invention to provide a reference non-linear dielectric spectrum: the apparatus may be calibrated by techniques described hereinafter for a first subject, and then used for further subjects of the same general type.

The apparatus according to the invention is preferably provided with means for obtaining a detectable signal. which may, for example. include a chart recorder. screen display, digital display or digital readout.

The mode of operation of apparatus according to the invention, in the method according to the invention, will now be described, by way of illustration, in more detail. When a field of appropriately low frequency is applied to cellular biological material contained between two or more macroscopic electrodes or within a coil, the charging of the membrane capacitance may cause an effective "amplification" of the macroscopic field across the membrane. In certain cases in which the membrane of interest contains appropriate enzymes, this can cause performance of useful biological work in a field- and frequency-dependent fashion. A general mechanism underlying this effect is that enzymes are not dipolar "billiard balls" and can relax between different conformations, some of which may and some of which may not have different vectorial dipole moments from each other.

The electrical potential applied to the biological material may comprise a high field applied to excite the material and a low probing AC voltage to register the field-dependent dielectric properties of the material.

It is preferred to use a sinusoidal AC field to excite the material and the entire frequency range of interest is observed by performing a transformation to see the extent to which the non-linearities of the biological material are manifest by the generation of harmonics. By varying the frequency and amplitude of the exciting current, a multi-dimensional non-linear dielectric spectrum can be built up which can act as a dielectric fingerprint of the sample under test.

Preferably the frequency of the excitation signal is below the range in which β-dispersion of the dielectric permittivity of the test material occurs. Typically therefore the excitation signal frequency is a maximum of 100 kHz (preferably a maximum of 1 kHz). Also, typically the excitation signal has a peak-to-peak value of 20 volts (preferably 4 volts): typically the outer electrodes are 2cm apart giving a field strength of ±5 volts/cm for a signal of 20 volts peak-to-peak (or ± 1 volt/cm for a signal of 4 volts peak-to-peak).

Membrane proteins (typically in living tissue) are particularly powerful candidates for interacting with electrical fields for a variety of reasons. including the following: (i) the membrane protein cannot rotate from one side of the membrane to the other and dissipate electrical energy by simple Debye-like rotation of this type; (ii) as described above, the membrane can "amplify" the exciting signal: and (iii) membrane proteins have substantial dipole moments. In addition, of course, in common with all proteins. they can effect transitions between different conformational states possessing different dipole moments. Thus in seeking a mechanistic basis for the remarkable generation of non-linear dielectric spectra that we have observed one is led to consider the membrane properties of cell material present in the biological material under test.

The apparatus is arranged to apply an electrical potential of one or more initial frequencies to the biological material, and to measure the response of the material at at least one response frequency substantially absent from (substantially not overlapping with) the initial frequency or frequencies. In some embodiments, the electrical potential comprises a high field applied to excite the system, and a low probing AC voltage to register the field-dependent dielectric properties of the biological material.

In the apparatus according to the invention, the abovementioned potential is preferably sinusoidal. and the entire frequency range of interest is preferably observed by performing a translation or vector transformation to ascertain the extent to which the non-linearities of the material are manifest by the generation of harmonics.

Other features of the apparatus according to the invention, and its mode of use may be substantially as described in our prior G.B-A-2247530, as referred to above.

Features of the invention will now be described, by way of example only, with reference to the accompanying drawings, in which:

> Figure 1 shows, schematically, certain features of exemplary apparatus according to the invention; and
> Figures 2 to 7 show results achieved in exemplary analyses using method and apparatus according to the invention.

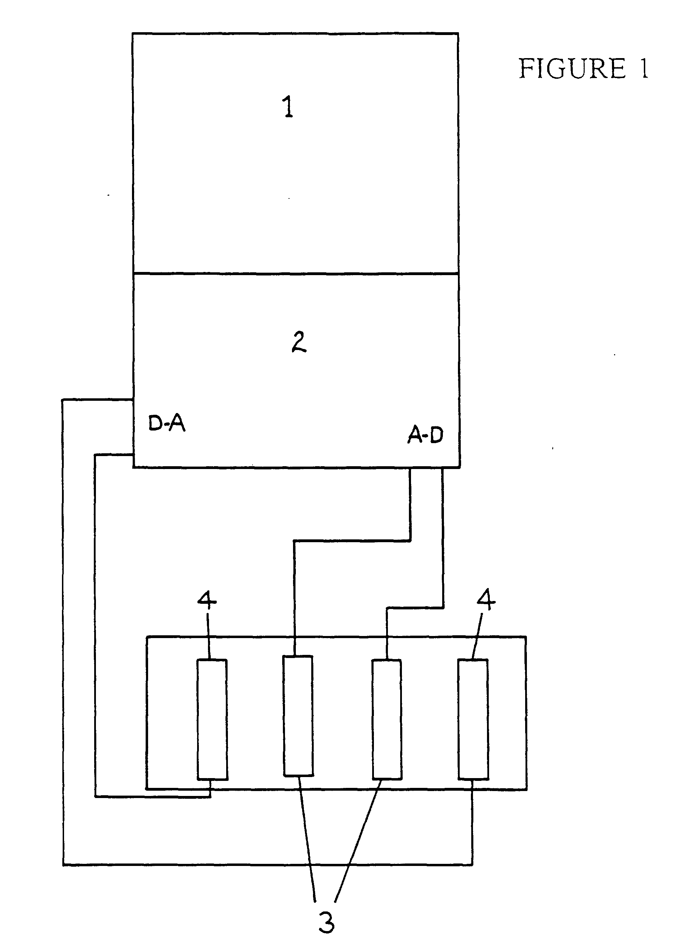

In the embodiment shown in Figure 1, an AC potential of predetermined frequency is applied by generator 2 via digital-to-analogue conversion D-A between an outer pair of electrodes 4 in order to excite the system, and cause an alternating potential to arise between an inner pair of electrodes 3. The AC potential arising between the inner pair of electrodes includes harmonics of the excitation frequency. A computer 1 carries out a Fourier transformation on the signal received from the inner pair of electrodes via analogue-to-digital conversion A-D, to determine the power levels at the first five (for example) harmonics. The process is repeated with different voltages of the excitation signal, and then at different excitation frequencies.

The excitation signal mav consist of a sinusoidal waveform. Alternatively the excitation signal may consist of a high DC with a low AC component superimposed on it.

In order that features of the present invention may be more fully understood, the following examples are given by way of illustration only.

In the examples, non-linear dielectric spectroscopy was carried out largely as described in GB-A-2247530, using in this case a matrix of 5 voltages (zero-to-peak (as measured on the outer electrodes of apparatus as illustrated in Figure 1) and 9 frequencies (in Hz), as follows:

| | |
|---|---|
| 0.500000 | |
| 0.750000 | |
| 1.000000 | Voltages |
| 1.250000 | |
| 1.500000 | |
| 10.000000 | |
| 17.782794 | |
| 31.622777 | |
| 56.234133 | |
| | |
| 100.000000 | Frequencies |
| 177.827941 | |
| 316.227766 | |
| 562.341325 | |
| 1000.000000 | |

A sweep consisted of 45 individual spectra, averaging each for 10 blocks. Further sweeps were taken at appropriate intervals. The sampling rate at the inner electrodes was adjusted to be 16 times the value of the frequency applied, such that no windowing was needed and after (Fourier) transformation the power in each consecutive harmonic appears in each consecutive bin. To avoid the need for a reference run (without cells), the following procedure was adopted. The data matrix, consisting of the powers in each of the first 5 harmonics (including the fundamental) at each voltage and frequency, was subjected to multivariate calibration using the partial least squares (PLS) algorithm, fully cross-validated by the leave-one-out method. Such multivariate calibrations are well known to those skilled in the chemometric art.

Example

A spot was marked on a human subject's forearm to ensure repeatable placement of a probe with flush electrodes on subsequent sweeps. The probe was also marked to ensure repeatability of orientation. Before each spectrum was taken, the probe was moistened in 150 mM NaCl to ensure good electrical coupling.

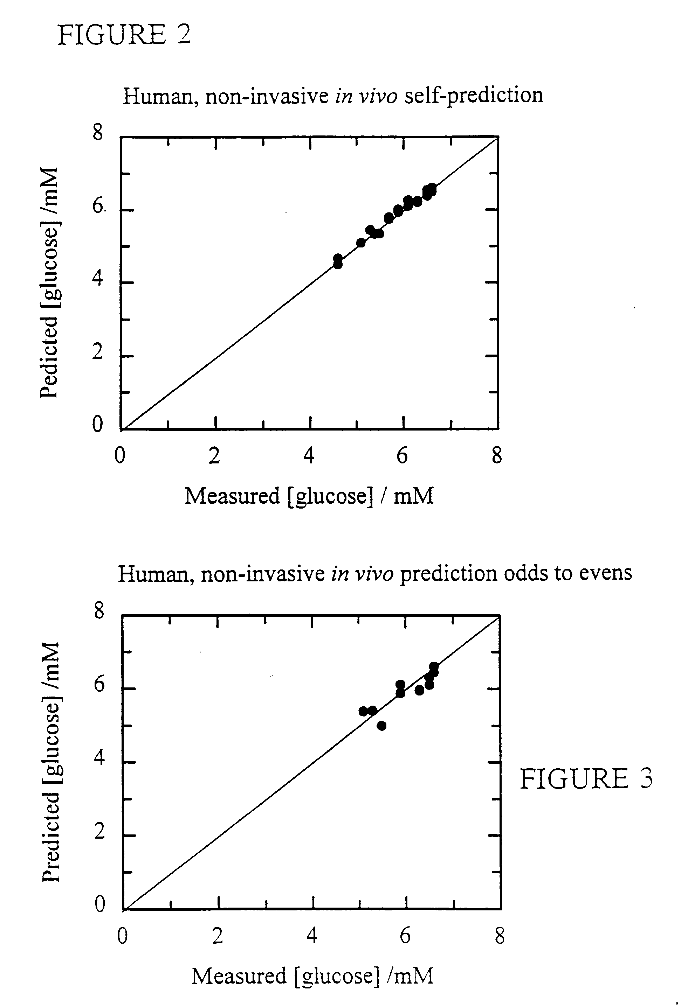

The first two experiments (3 figures) were carried out as follows. Baseline sweeps were taken after the subject had eaten no food for 16 hours, using the same voltages and frequencies as above. Glucose measurements were taken on finger-pricked blood with an optical blood glucose monitoring instrument commercially available under the trade mark "Reflolux" as the reference method. About 100g of glucose were given orally, and further sweeps taken at approx one minute intervals, checking with the Reflolux instrument every 5 minutes and interpolating these reference readings. To improve the ability of the calibration models to generalise, an iterative method for removing outliers was performed, as follows. First the data from a given run (run 1, me8) were used to make the best model, as judged by cross-validation, leave-one-out self-prediction. The model was used to predict run 2 (me7), and then the points chosen that are closest (within 0.5 mM) to the 1:1 line and the others assumed to be real outliers, i.e. bad data. These "good points" were then used to make a new model, again the best as judged by cross-validated, leave-one-out self-prediction. Finally, a calibration model was formed on the first run with outliers removed according to the revised prediction from the second run. The data for the self model so formed, fully cross validated, using 2 PLS factors, are shown in Figure 2.

Figure 3 shows the predictions from a calibration model of the same data produced on alternate (odd-numbered) points predicting the even-numbered points from the same run.

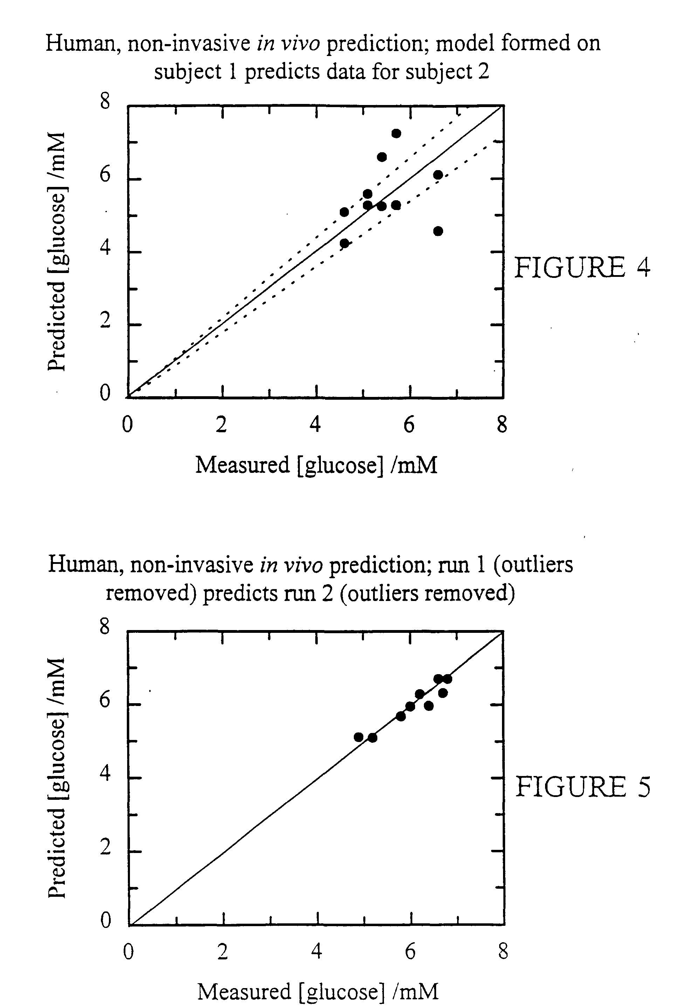

Figure 4 shows the prediction of the pruned dataset of me7 as predicted from the model formed on the pruned dataset of me8.

Data was acquired from a separate (and diabetic) subject, who had just eaten a meal, his blood glucose followed using the Reflolux instrument and non-linear dielectric spectra acquired exactly as above. The same calibration model as above (formed on the first subject) was used to predict blood glucose data from the second subject (when these were within the range that had been covered by the calibration model), as shown in Figure 5 (in which the dotted lines show accuracies of $\pm$ 10%, the claimed best precision of the reference method).

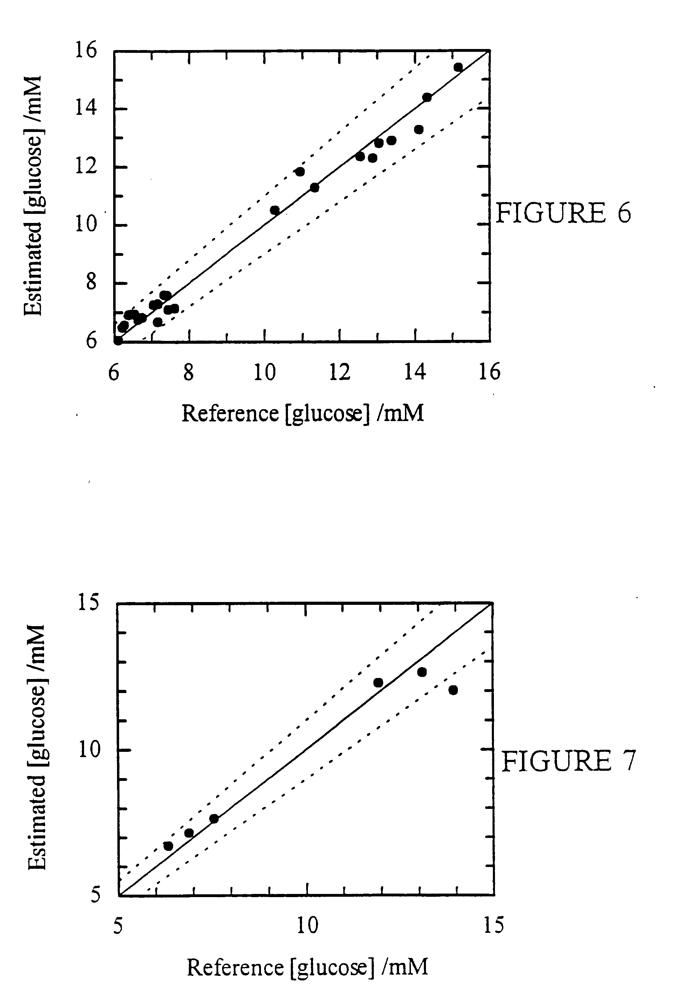

Finally, a combined model was produced for a separate pair of subjects (one diabetic, one non-diabetic). Figure 6 shows the self-calibration, fully cross-validated, using 5 PLS factors, whilst Figure 7 shows the predictions using data from the same subjects but which had not been included in the calibration model. In each case, the solid line is the line of identity whilst the dotted lines are identity ± 10%. This shows that the method according to the invention has excellent predictive power.

### Claims

1. Apparatus for monitoring or analysing a determinand associated with cellular biological material, which apparatus comprises:

    (a) means for applying an AC electrical potential at one or more discrete frequencies to said material;
    (b) means for determining a response of said material at one or more frequencies which were substantially absent from the applied AC potential; and
    (c) means for comparing said response to a calibration model of said determinand.

2. Apparatus according to claim 1, which further comprises means for retaining said biological material in proximity to said electric potential applying means.

3. Apparatus according to claim 2, wherein said means for retaining the biological material comprises adhesive provided on a patch or the like for retention of an electrode to a subject's skin.

4. Apparatus according to any of claims 1 to 3, wherein the electrical potential comprises a high field to excite the biological material, and a low probing AC voltage to register the field-dependent dielectric properties of the biological material.

5. Apparatus according to any of claims 1 to 4, wherein said potential is sinusoidal, and the apparatus is arranged to scan the entire frequency range of interest by performing a translation or vector transformation to ascertain the extent to which a non-linear dielectric response is manifest by the generation of harmonics.

6. Apparatus according to any of claims 1 to 5, wherein said response comparing means comprises data processing apparatus previously calibrated with a characteristic of cellular biological material of the same general type.

7. Apparatus according to any of claims 1 to 6, which further comprises means for obtaining a detectable signal depending on the results of comparison of said response to said stored characteristic.

8. A method of analysing or monitoring a determinand associated with cellular biological material such as a glucose concentration, which comprises applying an AC electrical potential at one or more discrete initial frequencies to a sample of said material; measuring a response of the material at at least one response frequency substantially not overlapping with said applied AC potential; and comparing said response with a stored calibration model of said determinand.

9. A method according to claim 8, wherein the electrical potential comprises a relatively high field to excite the biological material, and a relatively low probing AC voltage to register the field-dependent dielectric properties of the biological material.

10. A method according to claim 8 or 9, wherein said potential is sinusoidal, and the apparatus is arranged to scan the entire frequency range of interest by performing a translation or vector transformation to ascertain the extent to which a non-linear dielectric response is manifest by the generation of harmonics.

### Patentansprüche

1. Vorrichtung zum Überwachen oder Analysieren eines Parameters von zellulärem biologischen Material wobei die Vorrichtung umfasst:

    (a) Mittel zum Anlegen einer elektrischen Wechselspannung mit einer oder mehreren diskreten Frequenzen an das Material;

    (b) Mittel zum Bestimmen einer Reaktion des Materials bei einer oder mehreren Frequenzen, die in der angelegten Wechselspannung im Wesentlichen nicht enthalten sind, und

    (c) Mittel zum Vergleichen der Reaktion mit einem Kalibrierungsmodell des Parameters.

2. Vorrichtung nach Anspruch 1, die ferner Mittel zum Halten des biologischen Materials in der Nähe des Mittels zum Anlegen des elektrischen Potentials aufweist.

3. Vorrichtung nach Anspruch 2, wobei das Mittel zum Halten des biologischen Materials Klebemittel aufweist, die auf einem Flicken o.dgl. bereitgestellt sind, um eine Elektrode auf einer Haut eines Versuchsobjekts zu halten.

4. Vorrichtung nach einem der Ansprüche 1 bis 3, wobei die elektrische Spannung ein starkes Feld zum Anregen des biologischen Materials und eine schwache Mess-Wechselspannung aufweist, um die feldabhängigen dielektrischen Eigenschaften des biologischen Materials zu registrieren.

5. Vorrichtung nach einem der Ansprüche 1 bis 4, wobei die Spannung sinusförmig ist und die Vorrichtung angeordnet ist, den gesamten Frequenzbereich von Interesse abzutasten, indem eine Translation oder Vektortransformation durchgeführt wird, um das Ausmaß der nicht-linearen dielektrischen Reaktion festzustellen, welche sich durch die Erzeugung von Oberschwingungen zeigen.

6. Vorrichtung nach einem der Ansprüche 1 bis 5, wobei das Mittel zum Vergleichen der Reaktion eine Datenverarbeitungsvorrichtung aufweist, die zuvor mit einer Charakteristik von zellulärem biologischen Material derselben Art kalibriert worden ist.

7. Vorrichtung nach einem der Ansprüche 1 bis 6, die ferner ein Mittel zum Erhalten eines erfassbaren Signals in Abhängigkeit von den Ergebnissen des Vergleichs der Reaktion mit der gespeicherten Charakteristik aufweist.

8. Verfahren zum Analysieren oder Überwachen eines Parameters, von zellulärem biologischen Material wie beispielsweise einer Glukosekonzentration, mit den Schritten, eine elektrische Wechselspannung mit einer oder mehreren diskreten Anfangsfrequenzen an eine Probe des Materials anzulegen; eine Reaktion des Materials bei zumindest einer Reaktionsfrequenz zu messen, die mit der angelegten Wechselspannung im Wesentlichen nicht überlappt; und die Reaktion mit einem gespeicherten Kalibrationsmodell des Parameters zu vergleichen.

9. Verfahren nach Anspruch 8, wobei die elektrische Spannung ein relativ starkes Feld zum Anregen des biologischen Materials und eine relativ schwache Mess-Wechselspannung zum Registrieren der feldabhängigen dielektrischen Eigenschaften des biologischen Materials aufweist.

10. Verfahren nach Anspruch 8 oder 9, wobei die Spannung sinusförmig ist und die Vorrichtung angeordnet ist, den gesamten Frequenzbereich von Interesse abzutasten, indem eine Translation oder Vektortransformation durchgeführt wird, um das Ausmaß einer nicht-linearen dielektrischen Reaktion zu erfassen, die sich durch die Erzeugung von Oberschwingungen zeigt.

**Revendications**

1. Appareil de contrôle ou d'analyse d'une grandeur associée à de la matière biologique cellulaire, lequel appareil comprend :

    (a) des moyens pour appliquer un potentiel électrique alternatif à une ou plusieurs fréquences discrètes à ladite matière;
    (b) des moyens pour déterminer une réponse de ladite matière à une ou plusieurs fréquences qui étaient sensiblement absentes du potentiel alternatif appliqué ; et
    (c) des moyens pour comparer ladite réponse à un modèle d'étalonnage de ladite grandeur.

2. Appareil selon la revendication 1, qui comprend en outre des moyens pour maintenir ladite matière biologique à proximité desdits moyens pour appliquer le potentiel électrique.

3. Appareil selon la revendication 2, dans lequel les moyens pour maintenir la matière biologique comprennent un adhésif prévu sur un patch ou similaire pour le maintien d'une électrode sur la peau d'un sujet.

4. Appareil selon l'une quelconque des revendications 1 à 3, dans lequel le potentiel électrique comprend un champ élevé pour exciter la matière biologique, et une faible tension alternative de mesure pour enregistrer les propriétés diélectriques, dépendant du champ, de la matière biologique.

5. Appareil selon l'une quelconque des revendications 1 à 4, dans lequel ledit potentiel est sinusoïdal, et l'appareil est préparé pour balayer la plage complète de fréquences d'intérêt en réalisant une translation ou transformation vectorielle pour vérifier le degré auquel une réponse diélectrique non linéaire est le fait. de la génération d'harmoniques.

6. Appareil selon l'une quelconque des revendications 1 à 5, dans lequel lesdits moyens pour comparer la réponse comprennent un appareil de traitement de données préalablement étalonné avec une caractéristique de matière biologique cellulaire du même type général.

7. Appareil selon l'une quelconque des revendications 1 à 6, qui comprend en outre des moyens pour obtenir un signal détectable dépendant des résultats de comparaison de ladite réponse à ladite caractéristique stockée.

8. Procédé d'analyse ou de contrôle d'une grandeur associée à de la matière biologique cellulaire, telle qu'une concentration de glucose, qui comprend l'application d'un potentiel électrique alternatif à une ou plusieurs fréquences initiales discrètes à un échantillon de ladite matière ; la mesure d'une réponse de la matière à au moins une fréquence de réponse ne se chevauchant sensiblement pas avec ledit potentiel alternatif applique ; et la comparaison de ladite réponse à un modèle d'étalonnage stocké de ladite grandeur.

9. Procédé selon la revendication 8, dans lequel le potentiel électrique comprend un champ relativement élevé pour exciter la matière biologique, et une relativement faible tension alternative de mesure pour enregistrer les propriétés diélectriques, dépendant du champ, de la matière biologique.

10. Procédé selon la revendication 8 ou 9, dans lequel ledit potentiel est sinusoïdal, et l'appareil est préparé pour balayer la plage complète de fréquences d'intérêt en réalisant une translation ou transformation vectorielle pour vérifier le degré auquel une réponse diélectrique non linéaire est le fait de la génération d'harmoniques.

FIGURE 1

## FIGURE 2

Human, non-invasive *in vivo* self-prediction

Human, non-invasive *in vivo* prediction odds to evens

FIGURE 3

Human, non-invasive *in vivo* prediction; model formed on
subject 1 predicts data for subject 2

FIGURE 4

Human, non-invasive *in vivo* prediction; run 1 (outliers
removed) predicts run 2 (outliers removed)

FIGURE 5

FIGURE 6

FIGURE 7